# EUROPÄISCHE PATENTSCHRIFT

(11) **EP 1 926 696 B1**
(45) Veröffentlichungstag und Bekanntmachung des Hinweises auf die Patenterteilung: **29.04.2009**
(21) Anmeldenummer: 06792973.7
(22) Anmeldetag: 23.08.2006
(51) Int. Cl.: C07C 29/17, C07C 29/76, C07C 31/30, C07C 33/035

(54) **VERFAHREN ZUR ABTRENNUNG VON POLYMEREN NEBENPRODUKTEN AUS 1,4-BUTINDIOL**
METHOD FOR THE SEPARATION OF POLYMERIC BY-PRODUCTS FROM 1,4-BUTYNEDIOL
PROCEDE DE SEPARATION DE SOUS-PRODUITS POLYMERIQUES DU 1,4-BUTINDIOL

(30) Priorität: 06.09.2005 DE 102005042185
(43) Veröffentlichungstag der Anmeldung: 04.06.2008
(73) Patentinhaber: BASF SE, 67056 Ludwigshafen (DE)
(72) Erfinder: LORENZ, Rudolf Erich, 67063 Ludwigshafen (DE); PINKOS, Rolf, 67098 Bad Dürkheim (DE); STEINIGER, Michael, 67435 Neustadt (DE); SCHÄFER, Gerd, 67590 Monsheim (DE)
(86) Internationale Anmeldenummer: PCT/EP2006/065595
(87) Internationale Veröffentlichungsnummer: WO 2007/028716

(56) Entgegenhaltungen:
- EP-A2- 1 207 146
- DE-A1- 4 220 239
- US-A- 5 068 468

## Beschreibung

Die Erfindung betrifft ein Verfahren zur Reinigung von 1,4-Butindiol durch Abtrennung von Verunreinigungen in Form polymerer Nebenprodukten und von Katalysatorbestandteilen sowie ein Verfahren zur Herstellung von 1,4-Butendiol und 1,4-Butandiol durch Hydrierung des gereinigten 1,4-Butindiols, welches die Abtrennung polymerer Nebenprodukte umfasst.

Die Synthese von 1,4-Butindiol aus Acetylen und Formaldehyd wird industriell mehrfach betrieben und ist beispielsweise bei K. Weissermel, H.-J. Arpe, Industrielle organische Chemie, 5. Auflage, 1998, Wiley-VCH, Seiten 110 und 111) beschrieben worden. Neben Kupfer können die üblicherweise verwendeten Katalysatoren gegebenenfalls Wismut sowie Silikate (im Folgenden SiO₂ genannt) oder Aluminiumoxid enthalten. Während der Synthese von 1,4-Butindiol kommt es als Nebenreaktion zur Bildung von oligomerer beziehungsweise polymerer Substanzen (im Folgenden Cuprene genannt). Diese Cuprene gelangen üblicherweise zusammen mit löslichen und unlöslichen Bestandteilen des verwendeten Katalysators in die Hydrierstufe, bei der zunächst 1,4-Butendiol gebildet wird, das sich in einem weiteren Hydrierschritt zum gegenüber 1,4-Butendiol bedeutsameren Zwischenprodukt 1,4-Butandiol hydrieren lässt.

Die Hydrierung von 1,4-Butindiol zu 1,4-Butandiol wird seit Jahrzehnten betrieben und ist vielfältig beschrieben. So ist aus der US-A 5 068 468 ist die Hydrierung von 1,4-Butandiol an festen geträgerten Nickel-Kupfer-Katalysatoren bekannt, während US-A 4 153 578 ein zweistufiges Verfahren zur Hydrierung von 1,4-Butindiol an suspendierten Raney-Nickel-Molybdän-Katalysatoren bei einem Druck von 21 bar beschreibt. Die DD-A 272 644 offenbart die Suspensionshydrierung von wässrigem Butindiol an Nickel-SiO2-Katalysatoren und aus der EP-B 0 319 208, der DE-A 19 41 633 und der DE-A 20 40 501 sind allgemeine, unter anderem auf 1,4-Butindiol anwendbare, Hydrierverfahren bekannt.

Cuprene und Katalysatorbestandteile aus der Butindiolsynthese stören dessen Hydrierung zu 1,4-Butendiol oder 1,4-Butandiol und verschlechtern das Hydrierergebnis deutlich. So lagern sich Cuprene auf dem Katalysator ab und behindern den Kontakt von Butindiol mit der Katalysatoroberfläche mit der Folge, dass die Reaktion langsamer wird. Katalysatorkomponenten wie Kupfer, Wismut und/oder SiO₂ scheiden sich ebenfalls auf Katalysator ab und verändern so die Katalysatoraktivität und Selektivität.

Auch ohne den Ausbau des Katalysators können diese nachteiligen Effekte an der Bildung des Nebenprodukts Butanol verfolgt werden, da dessen Bildung durch die vorstehend genannten Katalysatorgifte beschleunigt wird.

Die einfache Reinigung von 1,4-Butindiol, z.B. durch Filtration ist kaum möglich, da insbesondere die Cuprene und SiO₂ zum Teil kolloidal beziehungsweise feinst verteilt vorliegen und somit gängige Filter schnell verstopfen, so dass die Filter entweder ständig ausgetauscht oder aufwändig rückgespült werden müssen.

Aufgabe der Erfindung ist es, ein einfaches wirtschaftliches Verfahren zur Verfügung zu stellen, mit dem durch die Abtrennung der in der Hydrierstufe unerwünschten Komponenten aus 1,4-Butindiol ein gereinigtes 1,4-Butindiol zugänglich wird, aus dem durch Hydrierung 1.4-Butendiol bzw. 1,4-Butandiol in hoher Selektivität und mit guter Katalysatorstandzeit gewonnen werden kann.

Es wurde nun überraschend gefunden, dass ein Verfahren, bei dem man technisches 1,4-Butindiol in einer dynamischen Mischvorrichtung in einer Inertgas-Atmosphäre bei 25 bis 150°C, bevorzugt 70 bis 90°C, bei einem Geschwindigkeitsgefälle im radialen Spiel zwischen Rotor und Stator der Mischvorrichtung von mehr als 100.000 sec⁻¹ bearbeitet, bei Temperaturen von 25 bis 150°C die Phasentrennung abwartet und die untere Phase abtrennt, zu einer Abtrennung der in der Hydrierstufe unerwünschten Komponenten aus technischem 1,4-Butindiol führt und durch dessen Hydrierung 1,4-Butendiol und 1,4-Butandiol jeweils in hoher Selektivität und mit guter Katalysatorstandzeit zugänglich werden. Unter technischem 1,4-Butindiol wird in dieser Anmeldung aus Acetylen und Formaldehyd an sich bekannten, üblichen Katalysatoren hergestelltes 1,4-Butindiol verstanden, das nach seiner Synthese nicht beispielsweise durch Destillation gereinigt wurde und im allgemeinen 10 bis 70 Gew.-% Butindiol, bevorzugt 30 bis 60 Gew.-% Butindiol, 0,2 bis 4 Gew.-% Propinol 0,1 - 2 Gew.-% Formaldehyd und 1 bis 2000 ppm, bevorzugt 3 bis 500ppm, besonders bevorzugt 10 bis 200 ppm, Cuprene und Katalysatorbestandteile sowie < 0,1 % andere Verunreinigungsspuren enthält.

1,4-Butindiol wird bevorzugt bei einem Geschwindigkeitsgefälle von 130.000 bis 145.000 sec⁻¹ zwischen 5 Minuten und 1 h, bevorzugt von 10 Minuten bis 20 Minuten, bearbeitet.

Die Behandlung des 1,4-Butindiols bei dem erfindungsgemäßen Schergefälle kann in zur Einstellung derartigen Gescheindigkeitsgefälle geigneten handelsüblichen Mischvorrichtungen, insbesondere dynamischen Mischvorrichtungen, zum Herstellen eines flüssigen, gegebenenfalls gasförmige Bestandteile enthaltenden Gemischs aus wenigstens zwei, insbesondere chemisch reaktionfähigen Komponenten erfolgen, die eine Regelung zur Einstellung der Temperatur aufweisen. Das Butindiol wird im erfindungsgemäßen Verfahren vor der Mischkammer auf 25 bis 150°C, bevorzugt 70 bis 90°C erhitzt. Bevorzugt sind jedoch Mischvorrichtungen, die ein Geschwindigkeitsgefälle im radialen Spiel zwischen Rotor (Laufrad) und Gehäusewand von mehr als 100000 (10⁵) sec⁻¹ gewährleisten. Eine derartige Mischvorrichtung bzw. Mischkammer ist beispielsweise aus der DE-C 42 20 239 bekannt. Bei dieser Mischkammer handelt es sich um eine Mischvorrichtung zum Herstellen eines flüssigen, gegebenenfalls gasförmige Bestandteile enthaltenden Gemischs mit einer rotationssymmetrischen Mischkammer, die aus einer Umfangswand und zwei Stirnwänden gebildet ist, mit mindestens einer Auslassöffnung für das Gemisch in der Umlaufwand der Mischkammer und mit einem in der Mischkammer angeordneten, drehantreibbaren Rotor, wobei der Rotor an seinem Umfang gleichmäßig verteilt Kantenbrüche und an seinen Stirnwänden Ausnehmungen aufweist, die mit ringförmigen Kanälen in den Stirnwänden der Mischkammer Druckzellen ausbilden, wobei die Druckzellen über Durchgangsbohrungen in den Rotor miteinander verbunden sind. Die Offenbarung dieser DE-C 42 20 239 sei durch Zitat vollständig eingeschlossen (incorporated by reference). Durch eine geeignete Auslegung des Rotors (Laufrad) im Verhältnis zur inneren Gehäusewand kann bei dieser Mischkammer ein Geschwindigkeitsgefälle von mehr als 100000 (10⁵) sec⁻¹ erreicht werden.

Das Inertgas wird mit einem Druck von im Allgemeinen 1 bis 2 bar bevorzugt über einen Anschluss unmittelbar in die Mischkammer zudosiert und ist ausgewählt aus Stickstoff, Kohlendioxid, Helium, Neon, Argon, Krypton und Xenon oder deren Gemischen.

Bei der erfindungsgemäßen Temperatur der Scherung wird sodann die Phasentrennung in einer Beruhigungszone, in der keine Mischung mehr erfolgt, abgewartet. Dies kann in einem oder mehreren gesonderten Phasentrenngefäßen erfolgen, die so ausgelegt sein sollten, dass die Temperatur regelbar ist. Die Zeit bis zum Eintritt der Phasentrennung, beträgt im Allgemeinen zwischen einer Stunde (h) und 5 Stunden, bevorzugt zwischen 2 Stunden und 5 Stunden.

Während des Lagerns in der Beruhigungszone bilden sich zwei Phasen, die dann mit an sich für diesen Zweck bekannten Verfahren wie z.B. durch Dekantieren oder Zentrifugieren , bevorzugt durch Zentrifugieren mit handelsüblichen Zentrifugen, voneinander getrennt werden.

Die schwerere (untere) Phase enthält überwiegend die Cuprene sowie erhebliche Anteile von Kupfer-, Wismut- oder SiO₂- haltigen, unerwünschten Katalysator-Komponenten. Diese untere Phase wird entweder der Entsorgung wie z.B. Verbrennung zugeführt, kann zuvor aber noch z.B. destillativ behandelt werden, um Restanteile an 1,4-Butindiol abzutrennen und ins erfindungsgemäße Verfahren zurückzuführen. Die vom Cuprenen und unerwünschten Katalysatorbestandteilen weitgehend befreite obere Phase wird der Hydrierung zu 1,4-Butendiol oder 1,4-Butandiol zugeführt.

Das erfindungsgemäße Reinigungsverfahren kann diskontinuierlich oder voll kontinuierlich durchgeführt werden, wobei die kontinuierliche Fahrweise bevorzugt ist.

Gegenstand der vorliegenden Erfindung ist zudem ein Verfahren zur Hydrierung von 1,4-Butindiol zu 1,4-Butendiol und bevorzugt zu 1,4-Butandiol, bei dem nach dem erfindungsgemäßen Verfahren gereinigtes 1,4-Butindiol eingesetzt wird.

Die Hydrierung von 1,4-Butindiol ist an sich bekannt und erfolgt bevorzugt in der Flüssigphase an fest angeordneten und/oder suspendierten Katalysatoren. Die Hydrierung kann bis zum 1,4-Butandiol, aber auch nur bis zum 1,4-Butendiol durchgeführt werden.

Für die Hydrierung von gereinigtem 1.4 Butindiol werden solche Katalysatoren verwendet, die C-C-Dreifach- und -Doppelbindungen zu Einfachbindungen zu hydrieren vermögen. Sie enthalten in der Regel eines oder mehrere Elemente der I., VI., VII. oder VIII.-Nebengruppe des Periodensystems der Elemente, bevorzugt die Elemente Kupfer, Chrom, Molybdän, Mangan, Rhenium, Eisen, Ruthenium, Kobalt, Nickel, Platin und Palladium. Besonders bevorzugt verwendet man Katalysatoren, die mindestens ein Element ausgewählt aus Kupfer, Chrom, Molybdän, Eisen, Nickel, Platin und Palladium enthalten.

Der Metallgehalt dieser Katalysatoren liegt in der Regel zwischen 0,1 - 100 Gew.-%, bevorzugt 0,2 - 95 Gew.-%, besonders bevorzugt 0,5 - 95 Gew.-%.

Der Katalysators enthält bevorzugt zusätzlich mindestens ein Element ausgewählt aus den Elementen der II., III., IV. und VI.-Hauptgruppe, der II., III., IV. und V. Nebengruppe des Periodensystems der Elemente und der Lanthanoiden als Promotor zur Aktivitätssteigerung.

Der Promotorgehalt des Katalysators beträgt in der Regel bis 5 Gew.-%, bevorzugt 0,001 - 5 Gew.-%, besonders bevorzugt 0,01 - 3 Gew.-%.

Als Katalysatoren können Fällungs-, Träger-, oder Raney-Typ-Katalysatoren verwendet werden, deren Herstellung beispielsweise in Ullmanns, Encyclopädie der technischen Chemie, 4. Auflage, 1977, Band 13, Seiten 558-665 beschrieben ist.

Als Trägermaterialien können Aluminiumoxide, Titanoxide, Zirkoniumdioxid, Siliciumdioxid, Tonerden, z.B. Montmorillonite, Silikate wie Magnesium- oder Aluminiumsilikate, Zeolithe sowie Aktivkohlen verwendet werden. Bevorzugte Trägermaterialien sind Aluminiumoxide, Titandioxide, Siliciumdioxid, Zirkoniumdioxid und Aktivkohlen. Selbstverständlich können auch Mischungen verschiedener Trägermaterialien als Träger für im erfindungsgemässen Verfahren anwendbare Katalysatoren dienen.

Diese Katalysatoren können entweder als Katalysatorformkörper, beispielsweise als Kugeln, Zylinder, Ringe, Spiralen, oder in Form von Pulvern verwendet werden.

Als Raney-Typ-Katalysatoren sind beispielsweise Raney-Nickel, Raney-Kupfer, Raney-Kobalt, Raney-Nickel/Molybdän, Raney-Nickel/Kupfer, Raney-Nickel/Chrom, Raney-Nickel/Chrom/Eisen oder Rhenium-Schwamm geeignet. Raney-Nickel/Molybdän-Katalysatoren können beispielsweise nach dem in der US-A 4 153 578 beschriebenen Verfahren hergestellt werden. Diese Katalysatoren werden aber auch beispielsweise von der Firma Degussa, 63403 Hanau, Deutschland vertrieben. Ein Raney-NickelChrom-Eisen-Katalysator wird beispielsweise unter der Handelsbezeichnung Katalysator Typ 11 112 W® von der Firma Degussa vertrieben.

Bei der Verwendung von Fäll- oder Trägerkatalysatoren werden diese zu Beginn der Reaktion bei 150 und 500°C im Wasserstoff- bzw. Wasserstoff/Inertgas-Strom reduziert. Diese Reduktion kann direkt im Synthese-Reaktor durchgeführt werden. Falls die Reduktion in einem separaten Reaktor durchgeführt wird, können die Katalysatoren vor dem Ausbau bei 30°C mit sauerstoffhaltigen Gasgemischen oberflächlich passiviert werden. Die passivierten Katalysatoren können in diesem Fall im Synthese-Reaktor vor dem Einsatz in einem Stickstoff/Wasserstoffstrom bei 180°C aktiviert oder auch ohne Aktivierung eingesetzt werden.

Die Katalysatoren können im Festbett oder in Suspension eingesetzt werden. Wenn die Katalysatoren in Form eines Festbetts angeordnet sind, wird der Reaktor nicht in der üblichen Rieselfahrweise, sondern in einem aufwärts gerichteten Gleichstrom von Flüssigkeit und Gas so betrieben, dass die Flüssigkeit und nicht das Gas als kontinuierliche Phase vorliegt.

Suspendierte Katalysatoren werden mit einer Korngröße im Allgemeinen von 0,1 - 500 µm, bevorzugt 0,5 bis 200 µm, besonders bevorzugt 1 bis 100 µm eingesetzt.

Wird mit suspendierten Katalysatoren gearbeitet, so wird bei Verwendung von gepackten Blasensäulen ebenfalls mit einem aufwärts gerichteten Gleichstrom von Flüssigkeit und Gas so gearbeitet, dass die Flüssigkeit und nicht das Gas als kontinuierliche Phase vorliegt. Das Verhältnis von das Reaktionsgefäß verlassender zu zugeführter Gasmenge beträgt bei der Verwendung von Festbettreaktoren und bei der Verwendung von gepackten Blasensäulen mit einem im Reaktionsmedium suspendierten Katalysator 0,99:1 bis 0,4:1.

Das erfindungsgemäß bei Festbettreaktoren und bei im Reaktionsmedium suspendierten Katalysatoren in gepackten Blasensäulen einzuhaltende Verhältnis von das Reaktionsgefäß verlassender zu zugeführter Gasmenge lässt sich in einfacher Weise einstellen, indem man die entsprechende Menge Wasserstoff entweder als Frischgas dosiert, oder technisch bevorzugt, Kreisgas rückführt und nur den durch chemischen Verbrauch und Abgas bedingten Wasserstoffverlust durch Frischwasserstoff ergänzt.

Das molare Verhältnis von Wasserstoff zu 1,4-Butindiol im Reaktor beträgt mindestens 3:1,bevorzugt zwischen 4:1 und 100:1.

Das erfindungsgemäße Verfahren wird an Festbettkatalysatoren mit Kreisgasfahrweise durchgeführt, d.h. das den Reaktor verlassende Gas wird im Kreislauf, gegebenenfalls nach Ergänzung mit frischem Wasserstoff, über einen Verdichter in den Reaktor rückgeführt. Es ist möglich, die gesamte Kreisgasmenge oder eine Teilmenge davon über einen Treibstrahlverdichter zu führen. In dieser bevorzugten Ausführungsform wird der Kreisgasverdichter durch eine kostengünstige Düse ersetzt. Die Verdichtungsarbeit wird über die ebenfalls im Kreis geführte Flüssigkeit eingebracht. Die erforderliche Druckerhöhung der Flüssigkeit zum Betreiben des Treibstrahlverdichters beträgt etwa 3 bis 5 bar.

Für die Durchführung des erfindungsgemäßen Verfahrens mit einem im Reaktionsmedium suspendierten Katalysator sind Strahldüsenreaktoren, Rührkessel und Blasensäulen mit Packungen, welche eine Packungsoberfläche von mindestens 500, bevorzugt 1000 bis 2000 m²/m³ aufweisen, geeignet. Strahldüsenreaktoren können in verschiedenen Bauarten Anwendung finden, wenn sie durch einen ausreichend hohen Energieeintrag, der erfahrungsgemäß oberhalb von 2 kW/m³ liegt, den für die Erfindung wesentlichen hohen Stoffübergang von der Gasphase an die Flüssigkeit mit den suspendierten Katalysatorteilchen gewährleisten können. Besonders geeignet sind Strahldüsenreaktoren, die mit einem Impulsaustauschrohr ausgerüstet sind. Eine industriell verbreitete Bauform eines Strahldüsenreaktors ist beispielsweise der in der EP-A 0 419 419 beschriebene Reaktor. Bei Werten für den Energieeintrag von 3 bis 5 kW/m³ ist mit diesem Reaktor eine Abscheidung der Gasphase noch in einfachen Abscheidern möglich, ohne Zusatzapparate wie Schaumzentrifugen oder Zyklone, benutzen zu müssen.

Rührbehälter sind für die Durchführung des erfindungsgemäßen Verfahrens nur dann geeignet, wenn der Energieeintrag in einem Bereich von 2 bis 10 kW/m³ liegt.

Strahldüsenreaktoren mit suspendierten Katalysatoren benötigen volumenbezogene Energieeinträge von mehr als 2 kW/m³, bevorzugt 3 - 5 kW/m³.

1,4-Butandiol findet in der Technik in großen Mengen Anwendung zum Beispiel bei der THF-Herstellung oder als Diolkomponente in Polyestern.

### Beispiele

Das erfindungsgemäße Verfahren wird anhand der nachfolgenden Beispiele näher erläutert. Wenn nicht anders angegeben, wurde technisches 1,4-Butindiol in Form einer 54 gew.-%igen wässrigen Lösung eingesetzt. Die Prozentangaben der Reaktionsausträge in den Beispielen stellen; wenn nicht anders vermerkt, wasserfrei gerechnete Gewichtsprozente dar, die gaschromatographisch ermittelt worden sind.

### Vergleichsbeispiel : technisches 1,4-Butindiol

190 ml eines Ni-Katalysator gemäß Beispiel 1 der US 5 068 468 wurden in einen Hydrierrektor mit 1,2 m Länge eingefüllt. Technisches 1,4-Butindiol in Form einer 54 gew.-%igen wässrigen Lösung wurde in einer Zulaufmenge von 100g/h in den Reaktor gegeben. Bei einer Temperatur von 140°C wurde bei einem Wasserstoffdruck von 200 bar und einem Flüssigumlauf 800 ml/h wurde 2 Wochen hydriert.

Innerhalb des Versuchszeitraums (2 Wochen) war der Butindiolumsatz immer vollständig. Die durchschnittliche Zunahme an n-Butanol lag bei ca. 0,06 % pro Tag. Nach Ausbau des Katalysators fanden sich Feststoffablagerungen auf dem Katalysator. Die Ausbeute an 1,4-Butandiol, bezogen auf eingesetztes Butindiol, zu Beginn 98,3 Gew. %, verringerte sich jedoch jeden Tag um 0,06 -Gew.-%.

### Beispiel 1: Reinigung des 1,4-Butindiols

### Reinigung:

Technisches Butindiol in Form einer 54 gew.-%igen wässrigen Lösung wurde mit einer Mischvorrichtung gemäß Figur 1 der DE-C 42 20 239 mit einer Spaltweite von 0,05 mm und einem Laufraddurchmesser von 48 mm bei 80°C 10 Minuten geschert und anschließend bei 80°C 22 Stunden bis zur Phasentrennung beruhigt. Sodann wurde auf 25°C abgekühlt und durch Dekantieren die untere Phase abgetrennt. Das so gereinigte Butindiol dem Hydrierreaktor zugeführt.

### Hydrierung:

Die Hydrierung wurde analog dem Vergleichsbeispiel durchgeführt, mit dem Unterschied, dass das erfindungsgemäß vorgereinigtes 1,4-Butindiol eingesetzt wurde. Die durchschnittliche Zunahme an n-Butanol lag bei ca. nur 0,035 % pro Tag. Nach Ausbau des Katalysators fanden sich keine Ablagerungen. Die Ausbeute an 1,4-Butandiol, bezogen auf eingesetztes Butindiol, betrug zu Beginn 98,3 Gew.% und verringerte sich jeden Tag um 0,035 Gew.%.

## Patentansprüche

1. Verfahren zur Reinigung 1,4-Butindiol, **dadurch gekennzeichnet, dass** man 1,4-Butindiol in einer dynamischen Mischvorrichtung in einer Inertgas-Atmosphäre bei 25 bis 150°C bei einem Geschwindigkeitsgefälle im radialen Spiel zwischen Rotor und Stator der Mischvorrichtung von mehr als 100.000 sec⁻¹ bearbeitet, bei Temperaturen von 25 bis 150°C die Phasentrennung abwartet und die untere Phase abtrennt .

2. Verfahren nach Anspruch 1, **dadurch gekennzeichnet, dass** bei einem Schergefälle von 130.000 bis 145.000 sec⁻¹ bearbeitet wird.

3. Verfahren nach Anspruch 1 oder 2, **dadurch gekennzeichnet, dass** bei 70 bis 90°C geschert wird.

4. Verfahren nach einem der Ansprüche 1 bis 3, **dadurch gekennzeichnet, dass** 5 Minuten bis 1 Stunde geschert wird.

5. Verfahren nach einem der Ansprüche 1 bis 4 , **dadurch gekennzeichnet, dass** das Inertgas ausgewählt ist aus Stickstoff, Kohlendioxid, Helium, Neon, Argon, Krypton, Xenon oder deren Gemisch.

6. Verfahren nach einem der Ansprüche 1 bis 5, **dadurch gekennzeichnet, dass** die Einstellung der Phasentrennung in einer Beruhigungszone erfolgt.

7. Verfahren nach einem der Ansprüche 1 bis 6, **dadurch gekennzeichnet, dass** aus der abgetrennten unteren Phase 1,4-Butindiol gewonnen und in das Reinigungsverfahren zurückgeführt wird.

8. Verfahren zur katalytischen Hydrierung von 1,4-Butindiol , **dadurch gekennzeichnet dass** 1,4-Butindiol nach einem der Ansprüche 1-7 gereinigt und dann hydriert wird.

9. Verfahren nach Anspruch 8 zur Herstellung von 1,4-Butendiol.

10. Verfahren nach Anspruch 8 zur Herstellung von 1,4-Butandiol.

## Claims

1. A process for purifying 1,4-butynediol, which comprises processing 1,4-butynediol in a dynamic mixing apparatus in an inert gas atmosphere at from 25 to 150°C at a shear rate in the radial gap between rotor and stator of the mixing apparatus of more than 100 000 sec⁻¹, awaiting phase separation at temperatures of from 25 to 150°C and separating off the bottom phase.

2. The process according to claim 1, wherein processing is carried out at a shear rate of from 130 000 to 145 000 sec⁻¹.

3. The process according to claim 1 or 2, wherein shearing is carried out at from 70 to 90°C.

4. The process according to any of claims 1 to 3, wherein shearing is carried out for from 5 minutes to 1 hour.

5. The process according to any of claims 1 to 4, wherein the inert gas is selected from among nitrogen, carbon dioxide, helium, neon, argon, krypton, xenon and mixtures thereof.

6. The process according to any of claims 1 to 5, wherein phase separation occurs in a calming zone.

7. The process according to any of claims 1 to 6, wherein 1,4-butynediol is recovered from the bottom phase which has been separated off and is recirculated to the purification process.

8. A process for the catalytic hydrogenation of 1,4-butynediol, wherein 1,4-butynediol is purified according to any of claims 1-7 and is then hydrogenated.

9. The process according to claim 8 for preparing 1,4-butenediol.

10. The process according to claim 8 for preparing 1,4-butanediol.

## Revendications

1. Procédé de purification de 1,4-butynediol, **caractérisé en ce que** le 1,4-butynediol est traité dans un dispositif de mélange dynamique dans une atmosphère de gaz inerte à 25 à 150 °C avec un gradient de vitesse dans l'espace radial entre le rotor et le stator du dispositif de mélange de plus de 100 000 sec⁻¹, la séparation des phases est attendue à des températures de 25 à 150 °C et la phase inférieure est séparée.

2. Procédé selon la revendication 1, **caractérisé en ce que** le traitement est réalisé à un gradient de cisaillement de 130 000 à 145 000 sec⁻¹.

3. Procédé selon la revendication 1 ou 2, **caractérisé en ce que** le cisaillement est réalisé à 70 à 90 °C.

4. Procédé selon l'une quelconque des revendications 1 à 3, **caractérisé en ce que** le cisaillement est réalisé pendant 5 minutes à 1 heure.

5. Procédé selon l'une quelconque des revendications 1 à 4, **caractérisé en ce que** le gaz inerte est choisi parmi l'azote, le dioxyde de carbone, l'hélium, le néon, l'argon, le krypton, le xénon ou leurs mélanges.

6. Procédé selon l'une quelconque des revendications 1 à 5, **caractérisé en ce que** la séparation des phases est réalisée dans une zone de détente.

7. Procédé selon l'une quelconque des revendications 1 à 6, **caractérisé en ce que** le 1,4-butynediol est obtenu à partir de la phase inférieure séparée et recyclé dans le procédé de purification.

8. Procédé d'hydrogénation catalytique de 1,4-butynediol, **caractérisé en ce que** du 1,4-butynediol est purifié selon l'une quelconque des revendications 1 à 7, puis hydrogéné.

9. Procédé selon la revendication 8 pour la fabrication de 1,4-butènediol.

10. Procédé selon la revendication 8 pour la fabrication de 1,4-butanediol.
